(19) Europäisches Patentamt
European Patent Office
Office européen des brevets

(11) EP 2 615 093 A1

(12) EUROPEAN PATENT APPLICATION

(43) Date of publication:
17.07.2013 Bulletin 2013/29

(51) Int Cl.:
*C07D 493/04* (2006.01)

(21) Application number: 12151277.6

(22) Date of filing: 16.01.2012

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **BIOeCON International Holding N.V.**
**Curaçao (AN)**

(72) Inventors:
- **Van Den Bergh, Johan**
  **3315 MA Dordrecht (NL)**
- **Moulijn, Jacob Adriaan**
  **2517 HB DEN HAAG (NL)**

(74) Representative: **Derks, Wilbert et al**
**Hoyng Monegier LLP**
**Rembrandt Tower, 31st Floor**
**Amstelplein 1**
**1096 HA Amsterdam (NL)**

# (54) Isolation of anhydro sugar hexitols by selective adsorbents

(57) A method is disclosed for recovering anhydrosugar alcohols from a mixture comprising closely related compounds, such as sugar alcohols. In the method the mixture is contacted with an adsorbent, whereby the anhydrosugar alcohols are selectively adsorbed. The anhydrosugar alcohols can be recovered by desorption from the adsorbent, using a desorbing solvent.

EP 2 615 093 A1

## Description

BACKGROUND OF THE INVENTION

1. Field of the Invention

The invention relates generally to a process to purify a mixture of anhydro sugar alcohols using hydrophobic adsorbents and more particularly to the recovery and purification of dianhydro sugar alcohols like isosorbide.

2. Description of the Related Art

Dianhydro sugar alcohols, such as isosorbide, isoidide and isomannide, and anhydro xylitol, are a class of renewable platform chemicals. These chemicals, or derivatives thereof, can be used to make polymers, pharmaceuticals and fuel additives.

The (di)anhydro sugar alcohols are commonly made via dehydration of sugar hexitols or pentitols using an acid catalyst. Typical catalysts include homogenous (e.g. hydrochloric acid, sulfuric acid, zinc chloride and copper chloride) and heterogeneous acid catalysts (e.g. proton exchanged zeolites, Amberlist and Nafion type polymers). The product mixture of this dehydration reaction consists of the unreacted sugar hexitols or pentitols, various anhydro sugar hexitols or pentitols, dianhydro sugar hexitols, oligomers, polymers, char, water and, in case of a homogeneous catalyst, the acid catalyst.

In order to obtain high purity (di)anhydro sugar hexitols or pentitols, they need to be isolated from the above described mixture.

Several methods to recover and purify isosorbide are known.

In US Patent No. 6,864,378 to Bhatia, the production of isosorbide is described by means of reactive distillation. Isosorbide is more volatile than sorbitol and the anhydro sorbitols and can be removed in this way.

US Patent No. 7,439,352 to Moore et al. describes a process that involves recovery of isosorbide using (wiped) film evaporators.

For this type of separation a point of concern is the limited thermal stability of isosorbide, which puts a constraint on the operating temperature. Therefore, the Moore et al. process is operated under high vacuum, which is economically unattractive. Moreover, even at reduced temperature product loss in the separation step still appears a great risk.

WO 00/14081 describes a process wherein the product isosorbide is removed in situ from the dehydration reaction using an organic solvent. O-xylene is claimed to selectively dissolve isosorbide over sorbitan and sorbitol. Clearly, addition of organic solvents brings along economic and environmental drawbacks.

In US Patent No. 4,564,692 to Feldmann, et al., crystallization is used to obtain isosorbide from an anhydro sugar alcohol mixture.

Melt crystallization is also mentioned as purification step for isosorbide in US patent 7,439,352.

However, in the process of US patent 4,564,692, the initial solution needs to contain at least 40% of the desired anhydro sugar alcohol. This method leads to relatively low single step yields and requires work up of starting solutions when the organic content in the solution is low.

US patent 2,524,414 to Wolfrom et al. claims a method to separate carbohydrates using column chromatography. Isosorbide is one of the carbohydrates being considered.

WO patent application 02/39957A2 claims a process to recover isosorbide from crude dehydration reaction mixtures using chromatography. The separation can be carried out using zeolites and carbons, ion exchange resins being specifically claimed. Simulated Moving Bed is named as technology to perform this separation. A downside of working with ion exchange resins is that their stability is limited when a strong homogeneous acid is present in the reaction products, and that ionic species in the solution can ion-exchange with the resin which requires frequent regeneration of the column.

In addition to these chromatographic separations, adsorption is mentioned in several patents as part of the purification method. Contact of isosorbide mixtures with (activated) carbon can remove char, tar, polymers or oligomers from the solution. Contact with ion-exchange resins is known to remove residual acids or other ionic species from the solution.

It appears that vacuum distillation or evaporation is the most common method for use in a recovery and purification process of isosorbide from the dehydration mixtures. Prior art distillation patents always start from concentrated anhydrosugar alcohol solutions, since most dehydration processes start from a highly concentrated sorbitol solution.

Recently, also conversion routes starting with low sorbitol concentrations have been investigated (Almeida et al., ChemSusChem, 2010, 3, 325.). The process presented by Almeida et al. starts by dissolution of cellulose in a concentrated aqueous zinc chloride solution. This resulting solution contains less than 10 %w of (hydrolyzed) cellulose. The cellulose is hydrolyzed to glucose, then hydrogenated to sorbitol and, finally, dehydrated to isosorbide in the same

solvent. This leads to a dehydration product stream that contains less than 10%w of anhydrosugars in a concentrated aqueous zinc chloride solution.

For the type of solutions produced by the Almeida et al. process, distillation is not a suitable isosorbide recovery method. Firstly, due to the low concentration of isosorbide in the mixture, low partial pressures can be expected. Secondly, since water is much more volatile than isosorbide, water will be distilled off before isosorbide, which can lead to precipitation of zinc chloride in the still.

Hydrophobic adsorbents like zeolites, (activated) carbons and polymers are known to be able to remove organic molecules from aqueous solutions (e.g. US patent application publication No. 2005/0202139 A1, and US patent application publication No. 2005/0202141 A1).

Moreover, reverse osmosis and nano-filtration membranes are known to separate water from ionic species by size exclusion of the relative large hydrated ions. This could be a specific advantage to use adsorbents with small micropores when salts and/or acids are present in the solution.

Thus, there is a particular need for a process for recovery and purification of anhydrosugars from a crude dehydration mixture, particularly in case that the reaction mixtures contain these anhydrosugars together with significant amounts of inorganic salts and/or acids. In addition, such process is preferably carried out at low temperatures, to prevent degradation of isosorbide.

## BRIEF SUMMARY OF THE INVENTION

The present invention relates to a method for recovering an anhydrosugar alcohol from a mixture further containing the corresponding sugar alcohol, said method comprising the steps of

a. contacting the mixture with an adsorbent whereby the anhydrosugar alcohol is selectively adsorbed;

b. separating the adsorbent from the mixture;

c. desorbing the anhydrosugar alcohol from the adsorbent by contacting the adsorbent with a desorbing solvent.

In an optional subsequent step the anhydrosugar alcohol is isolated from the desorbing solvent.

The method can be used, for example, for isolating isosorbide from a mixture further containing sorbitol and sorbitan.

## BRIEF DESCRIPTION OF THE FIGURES

Figure 1A shows the selective adsorption of Isosorbide on various activated carbon adsorbents from an aqueous solution at 298 K. Solid, dashed and dash-dot lines represent data of ACW15, ACW35 and ACW90, respectively. (see Example 1)

Figure 1B shows the selective adsorption of Isosorbide on various zeolites (MFI-400, BEA-300 and USY-80) and Polystyrene copolymerized with divinylbenzene from an aqueous solution at 298 K. Solid, dashed, dash-dot and dotted lines represent data of BEA-300, USY-80, MFI-400, and PSDVB, respectively. (see Example 1)

Figure 2A shows the Isosorbide loading on two activated carbons and Polystyrene copolymerized with divinylbenzene as a function of the weight fraction of $ZnCl_2$ in solution at 298 K. The Isosorbide weight fraction in the solution is about 0.03-0.04. Solid, dashed and dotted lines represent data of ACW15, PSDVB and ACW35, respectively. (see Example 2)

Figure 2B shows the Isosorbide loading on several zeolites (MFI-400, BEA-300 and USY-80) as a function of the weight fraction of $ZnCl_2$ in solution at 298 K. The Isosorbide weight fraction in the solution is about 0.03-0.04. Solid, dashed and dash- dotted lines represent data of BEA-300, USY-80 and MFI-400, respectively. (see Example 2)

## DETAILED DESCRIPTION OF THE INVENTION

The following is a detailed description of the invention.

<u>Definitions</u>

The term "sugar alcohol" as used herein means a sugar-based alkanol. In the case sugars having 5 carbon atoms the sugar alcohol is a pentitol; sugar alcohols having 6 carbon atoms are known as hexitols. Examples of sugar alcohols include xylitol (1,2,3,4,5-pentahydroxypentane, a pentitol), and sorbitol (D-glucitol), which is a hexitol. Sugar alcohols typically have several chiral carbon atoms, so that several stereo-isomers exist.

The term "anhydrosugar alcohol" refers to a class of materials that can be obtained by partial or complete dehydration of sugar alcohols. A sugar alcohol from which one water molecule has been removed is referred to as a

monoanhydrosugar alcohol; a sugar alcohol from which two water molecules have been removed is referred to as a dianhydrosugar alcohol. As water molecules are removed from a sugar alcohol molecule, the molecule becomes progressively smaller and less hydrophilic. Isosorbide, which is a dianhydrosugar alcohol derived from sorbitol, is of particular economic interest. Sorbitol itself can be prepared by hydrogenation of glucose.

Anhydrosugar alcohols are generally prepared in a catalytic dehydration reaction of the corresponding sugar alcohols. Inorganic Brønstedt acids, such as hydrochloric acid and sulfuric acids, are often used as the acid catalyst. Inorganic Lewis acids, such as $ZnCl_2$, and combinations of inorganic Brønstedt and Lewis acids may also be used. A particularly suitable catalyst composition is a mixture of $ZnCl_2$ and HCl.

Dehydration of sorbitol leads to isosorbide. However, isosorbide has three stereo-isomers: Isosorbide, isomannide and isoidide. In general, isosorbide is of main interest. If sucrose is used as the starting material, however, sucrose is first converted to glucose and fructose. Fructose can be hydrogenated to mannitol, which can be dehydrated to isomannide. This leads to a mixture of isosorbide and Isomannide.

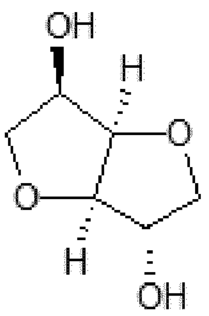

Isosorbide

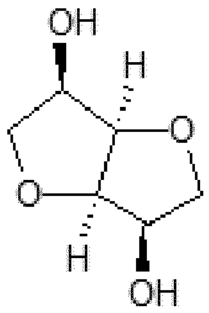

Isomannide

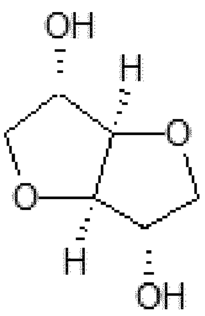

Isosidide

In general, the reaction product of a dehydration reaction of a sugar alcohol is an aqueous solution of the acid catalyst, unconverted sugar alcohol, and mono- and dianhydrosugar alcohols. Processes are needed for isolating the organic components of this mixture from the inorganic components, and for separating the dianhydrosugar alcohols from the unconverted sugar alcohol and the monoanhydrosugar alcohol.

The hydrophobic adsorbent can be, for example, a zeolite, a silica, or an activated carbon.

A zeolite is a crystalline aluminosillicate. A wide variety of zeolites exists, together with a wide variety of trivial names. We can classify zeolites in terms of a three letter code, as prescribed by the Structure Commission of the International Zeolite Association (IZA), and a silica to alumina molar ratio (SAR, Si/Al). The three letter code defines the zeolite topology. When the zeolite contains Aluminium, counterions are always present to balance the negative charge on the framework-aluminium. The pore size of a zeolite can be crudely classified according to the number of silicium and aluminium atoms in the ring that determines the pore size. We can distinguish zeolites with 8, 10, 12-membered-ring windows, also some larger pore zeolites have been recently discovered.

The hydrophobicity of a zeolite is strongly influenced by the silica to alumina ratio (SAR, Si/Al). Zeolites with very low SARs can be very hydrophilic and have a very strong water adsorption, whereas zeolites with very high SARs are very hydrophobic with very little water adsorption. The lower SAR limit is 1 (e.g. zeolite A, a form of LTA), the upper limit is an infinite SAR (e.g. silicalite-1, the all-silica form of MFI).

In this work we define a zeolite to be hydrophobic when the SAR $\geq$ 5; preferred for use in the present invention are zeolites having SAR $\geq$ 10.

Our findings demonstrate that Isosorbide can be adsorbed very well on hydrophobic forms of zeolite FAU, MFI and BEA. Note that FAU and BEA are zeolites with twelve-membered-ring pore-openings and that MFI has 10-membered-ring openings. This means that Isosorbide is able to enter even zeolites with 10-membered-ring pore-openings. Therefore, it is believed that any hydrophobic zeolite with a pore-opening of at least a 10-membered-ring will be useful for the separations in the process of the present invention.

Some common examples of such zeolite topologies are listed below (common trivial names of certain forms of the topology in brackets). This list is far from complete, for an exhaustive list of all known zeolite topologies see (Ch. Baerlocher and L.B. McCusker, Database of Zeolite Structures: http://www.iza-structure.org/databases/).

MFI(ZSM-5, Silicalite-1, Pentasil), MEL, MTW, LTL, FAU (Faujasite, Zeolite Y, Zeolite X, USY), MOR (Mordenite), FER (Ferrierite), BEA (Beta)

In addition to hydrophobic zeolites it has been found that activated carbons are able to adsorb isosorbide. Examples of carbon-based adsorbents that can useful for the process of the invention include (trivial or commercial

names between brackets):

Activated charcoal, Coconut Charcoal, activated carbon, porous graphitized carbon black (Carbopack or Carbotrap), Carbon Molecular sieves (Carboxen and Carbosieve series) and Sibunit.

Other suitable absorbents include silica-based materials. Silica is by nature very hydrophilic and used for desiccation purposes. However, by functionalizing silica can become more hydrophobic. Examples include : Octadecyl-functionalized silica gel or Phenyl-functionalized silica gel, available from SigmaAldrich.

The preferred adsorbent has a BET surface area of: 50 to 5000, preferably greater than 200, most preferably greater than 500, and a pore volume of 0.1 to 5 ml $g^{-1}$, preferably between 0.2 and 2.

The preferred adsorbent contains micro-pores with a preferred size between 0.4 and 100 nm, preferably between 0.5 and 10 nm, more preferably between 0.5 and 2 nm, most preferably between 0.5 and 1 nm.

In the application of selective capture of Isosorbide by molecular sieving and separation of Isosorbide isomers, the preferred micro-pore-size is between 0.5 and 0.7 nm and a selectivity of at least: 1.5, preferably above 5, most preferably above 10. The selectivity is defined as : $x_{w,j}/x_{w,i}$ $*q_i/q_j$. ($x_w$ is the mass fraction in the liquid phase, q is the loading on the sorbent in g per g of sorbent.)

Note that here the claimed micro-pore-size does not exclude the presence of larger pores like meso or macropores. It is obvious to people skilled with the art that the coexistence of larger pores can be highly beneficial for the mass transport in the adsorbent since diffusion in micro-pores can be very slow. Therefore, when the claimed adsorbents will be used in a fixed bed operation on an industrial scale, it will preferably contain both micro and larger pores. In case of activated carbons this can be manufactured by preparing such materials in the form of granular activated carbons. Zeolite powders can be shaped by processes like granulation, pressing or extrusion. Commonly used binders are clays, alumina or silica.

The preferred particle size of particles used in a packed bed application will be between 1 and 50000 micron, preferably between 10 and 50000, most preferably between 100 and 50000.

In its broadest aspect the present invention relates to a method for recovering an anhydrosugar alcohol from a mixture further containing the corresponding sugar alcohol, said method comprising the steps of

a. contacting the mixture with an adsorbent whereby the anhydrosugar alcohol is selectively adsorbed;

b. separating the adsorbent from the mixture;

c. desorbing the anhydrosugar alcohol from the adsorbent by contacting the adsorbent with a desorbing solvent.

Depending on the selected adsorbent and on the conditions used in step a., the selective adsorption can be adsorption of all organic components, separating them from the inorganic components, such as the acid catalyst(s); or it can be adsorption of anhydrosugar alcohols, separating the anhydrosugar alcohols from the inorganic components and from unconverted sugar alcohols; or it can be adsorption of dianhydrosugar alcohols, separating them from the inorganic components, unconverted sugar alcohol, and monoanhydrosugar alcohols.

It will be understood that the process can be carried out in two or more steps, for example a first step in which the organic components of the mixture are separated from the inorganic components; and a second step in which, for example, the organic components are separated into dianhydrosugar alcohols, on one hand, and a mixture of unconverted sugar alcohol and monoanhydrosugar alcohols on the other. If the desired end product is a dianhydrosugar alcohol, such as isosorbide, the mixture of unconverted sugar alcohol and monoanhydrosugar alcohols may be recycled to the dehydration reaction.

The selective adsorption can be based on differences in hydrophilicity between the various components of the reaction mixture; on differences in size between the molecules of the organic components of the reaction mixture; or on a combination of these two types of differences.

Thus, in one embodiment of the invention, anhydrosugars are isolated from crude reaction mixtures based on molecular sieve adsorbents. This separation works also when large amounts of a salt and/or strong acid are present in the crude reaction mixture. It is found, for example, that zeolite MFI selectively adsorbs isosorbide from a mixture of a hexitol, several sorbitans and isosorbide, water and a salt hydrate (e.g. zinc chloride hydrate). It appears that this separation is based on size differences.

The method of this embodiment comprises of the following steps:

a. The aqueous dehydration product solution containing anhydrosugars, with or without salt or acid, is contacted with a molecular sieve adsorbent. This leads to a selective adsorption of isosorbide.

b. The adsorbent is contacted with a solvent (desorbent) that promotes desorption of the adsorbed products. Here the adsorbed products, such as isosorbide, become dissolved in the desorbent.

c. The desorbed products are separated from the desorbent, e.g. by evaporation of the desorbent.

Examples of suitable desorbents include polar organic solvents, in particular water, and alcohols, such as methanol and ethanol.

In this process high purity isosorbide can be obtained in one step. It has been found that this method can be used to separate isosorbide from other dianhydrosugar alcohols, such as isomannide. The method can also be used to separate a pentitol, such as xylitol, from its anhydroxylitols.

Further treatment of the isosorbide may be required. This can be done by a second adsorption step or any other process known in the prior art: e.g. vacuum distillation or crystallization.

The purity of the isosorbide can also be increased by contacting the desorbed product with (macroporous) activated carbon or ion exchange resins, as described in the prior art.

People skilled with the art will recognize that this process can be carried out in a slurry, fixed bed, true moving bed or simulated moving bed type fashion.

In a second embodiment a crude reaction mixture, which comprises inorganic components such as a mineral acid and/or a salt, such as $ZnCl_2$, is contacted with a hydrophobic adsorbent. The organic components of the reaction mixture are selectively adsorbed to the hydrophobic adsorbent, and can be desorbed using a desorbent as described above. Examples of suitable hydrophobic adsorbents include activated carbon, in particular macroporous activated carbon; hydrophobic zeolites, such as zeolites having high Si/Al ratio; and the like.

The mixture of organic components obtained from this selective adsorption process can be further separated into, for example, dianhydrosugar alcohols on the one hand, and unconverted sugar alcohols and monoanhydrosugar alcohols on the other hand, for example using the method of the first embodiment of this invention.

DESCRIPTION OF ILLUSTRATIVE EMBODIMENTS/EXAMPLES

Abbreviations:

Isosorbide: 1,4-3,6-Dianhydrosorbitol

Sorbitans: collection of all possible monoanhydrosorbitol isomers

ACW15: Activated Carbon ‘W15’

ACW35: Activated Carbon ’W35’

ACW90: Activated Carbon ’W90’

$ZnCl_2$: Zinc Chloride

USY-80: Ultra stable Y zeolite with a Silica to Alumina Ratio (SAR) of 80

MFI-400: Zeolite Mobil Five with a SAR of 400

BEA-300: Zeolite Beta with a SAR of 400

PS-DVB stands for styrene-divinylbenzene copolymer.

14AG: 1,4-anhydroglucitol = 1,4-anhydrosorbitol

36AG: 3,6-anhydroglucitol = 3,6-anhydrosorbitol

14&36AG stands for 1,4 and 3,6-anhydro-D-glucitol

25AM: 2,5-anhydromannitol

TOC: total organic content

Hydrophobic adsorbent: Examples of hydrophobic adsorbents are (activated) carbons, polystyrene based polymers, types of silica and zeolites with a silica to alumina ratio of at least 10.

Sugar hexitol: Sorbitol, Mannitol....others

Anhydro sugar hexitols: Collection of mono and di-dehydrated sugar hexitols. In case of the sugar hexitol sorbitol these are for example, but is not limited to: 1,4-anhydro sorbitol, 1,5-anhydro sorbitol, 3,6-anhydro sorbitol, 2,5-anhydromannitol, Isosorbide, isomannide and, isoidide.

General methods

Table 6 shows a list of adsorbents.

Equilibrium adsorption experiments have been carried out. The following pretreatment steps have been applied for the different adsorbents. The activated carbon materials (ACW15, ACW35 and ACW90) were used as obtained. The styrene based polymer (PSDVB) was dried for several hours at 120 °C to remove all the water present in the supplied sample (~50%w). The zeolites (MFI-400, BEA-300 and USY-80) were thermally activated overnight at 600 °C in air before use.

All equilibrium adsorption data were measured by mixing a known solution with a known amount of adsorbent for at least 20 h at 298K, or as mentioned otherwise in the specific example. The mass fractions in the initial solution

($x_{w,in}$) and the mixture after the experiment ($x_{w,fin}$) were analyzed by high performance liquid chromatography (HPLC). The adsorbed amount (loading, q) was calculated under the assumption that the mass of bulk solution ($m_{mix}$) was unchanged during the experiment:

$$q_i\ [g/g]=(m_{mix}\ (x_{w,i,in}-x_{w,i,fin})/m_{ads}$$

The selectivity is defined as: $x_{w,j}/_{xw,i}$ $*q_i/q_j$. ($x_w$ is the mass fraction in the liquid phase, q is the loading on the sorbent in g per g of sorbent.)

*Table 1. Suppliers and key properties of the adsorbents used.*

| **Adsorbent** | **Supplier** | **Product name** | **$SiO_2/Al_2O_3$** | **BET surface area / $m^2\ g^{-1}$** | ***$d_{part}$* micron** | ***$d_{pore}$* nm** |
|---|---|---|---|---|---|---|
| (H-)BEA | Zeolyst | CP811C-300 | 300 | 700 | 1-2 | 0.75 |
| ($NH_4$-)MFI | Zeolyst | CBV-28014 | 400 | 400 | 0.5-1 | 0.55 |
| (H-)USY | Zeolyst | CBV-901 | 80 | 500 | 0.5-1 | 0.75 |
| PSDVB | Sigma Aldrich | 41,910-9 | - | - | 300-800 | 4.6 |
| ACW15 | Norit | | - | 1150 | 15[b] | ?[a] |
| ACW35 | Norit | | - | 1150 | 38 | ? |
| ACW90 | Norit | | - | 750 | >>18 | ? |
| MCM41 | Sigma Aldrich | N/A | infinite | ? | ? | ? |

[a]The exact pore size is not known, but due to the activation method AC W15 should be the most microporous and AC W90 the most mesoporous. [b]The carbon samples have a broad particle size distribution.

Example 1

For each adsorbent an adsorption isotherm of isosorbide in water was measured, the results are shown in **Figure 1.** It is evident that all adsorbents are able to adsorb isosorbide from an aqueous solution containing a relative low isosorbide weight fraction in the solution. The loading on the adsorbent varied from 6 (MFI-400) to almost 30 % by weight (ACW15).

This example shows that all hydrophobic adsorbents considered are able to adsorb isosorbide selectively from an aqueous solution.

Example 2

In this example adsorption equilibrium data of isosorbide at 298 K from solutions containing different $ZnCl_2$ concentrations were measured. **Figure 2** shows the effect of $ZnCl_2$ on the amount of isosorbide that was adsorbed. This has been considered at an isosorbide weight fraction in the solution of ~0.03-0.04. ACW15, ACW35 and PSDVB showed a strong decrease in loading of isosorbide with increasing $ZnCl_2$ weight fraction. The loading of isosorbide on Zeolite BEA-300 appears independent of $ZnCl_2$ concentration and in case of MFI-400 and USY-80 a small decrease in isosorbide loading is observed.

It is essential to mention at this point that the $ZnCl_2$ concentration in the solution did not change notably in case of the zeolites, indicating a very high isosorbide to $ZnCl_2$ adsorption selectivity. In case of the carbons and PSDVB, signs of a small $ZnCl_2$ concentration reduction were observed. The amount of $ZnCl_2$ adsorbed was low in all cases and difficult to quantify accurately, but the amount adsorbed decreased with increasing micro-porous character of the adsorbent.

This example shows that in the presence of zinc chloride adsorbents with small micropores (approx. < 1 nm) showed the best performance. The adsorbents with mesopores and or large micropores showed a strong reduction of the amount of isosorbide adsorbed. This can be explained by competitive adsorption effects by the (hydrated) salt species. In small micropores the hydrated ions can be excluded from the pores leading to little or no (competitive) adsorption of the salt or (hydrated) ionic species. A second explanation can be that in micropores adsorption of Isosorbide is stronger which reduces the negative effects induced by the $ZnCl_2$ presence.

Example 3

A mixture of 48% $ZnCl_2$, 2.3% isosorbide, 1.6% 14&36AG, 0.3% 25AM, 1.1% sorbitol and approximately 46% water (balance) was obtained by dehydration of sorbitol in a concentrated $ZnCl_2$ solution. The color of the solution was black, indicating the presence of an unknown amount of char or tar. The dehydration reaction was stopped at incomplete conversion to obtain the current mixture composition with a high concentration of unreacted sorbitol and 14&36AG.

This mixture was contacted with zeolite BEA-300 and ACW15 at several adsorbent to organic content ratios. In Table 2 the results are shown, normalized with respect to the initial concentration to reveal the selectivity of the adsorbents with respect to the various species in the system. In both systems the $ZnCl_2$ weight fraction was almost constant, or even slightly increasing, i.e. $ZnCl_2$ was not significantly adsorbed. The 2,5-anhydromannitol concentrations were low and should be treated with caution. In case of BEA-300, significant amounts of sorbitol and 1,4-Anhydrosorbitol were adsorbed. The order in weight fraction reduction in this system was: Isosorbide >> 1,4-Anhydrosorbitol > Sorbitol >> $ZnCl_2$. In case of ACW15 the differences between the different organic species was much smaller: Isosorbide ≥ 1,4-Anhydrosorbitol > Sorbitol >> $ZnCl_2$.

In all cases the Isosorbide/$ZnCl_2$ selectivity was very high (>>10). In Table 2 also the Isosorbide to 14&36AG and Sorbitol selectivities are shown. Clearly, BEA-300 has much higher Isosorbide selectivities than ACW15.
This example shows that in addition to the ability of hydrophobic adsorbents to adsorb all anhydro sugars from an aqueous zinc chloride solution, they are selective towards the more hydrophobic species.

*Table 2. Adsorption selectivity of ACW15 and zeolite BEA-300 in a dehydration product solution.*

| Adsorbent | Adsorbent/ solution Ratio g/g | fraction left in solution *($x/x_{in}$)* | | | | | Isosorbide selectivity | |
|---|---|---|---|---|---|---|---|---|
| | | $ZnCl_2$ | Isosorbide | 14& 36AG | 25AM | Sorbitol | 14& 36AG | Sorbitol |
| ACW15 | 0.078 | 0.99 | 0.73 | 0.76 | 0.86 | 0.87 | 1.1 | 2.0 |
| ACW15 | 0.16 | 1.00 | 0.50 | 0.60 | 0.82 | 0.74 | 1.3 | 1.9 |
| BEA-300 | 0.095 | 1.01 | 0.46 | 0.90 | 0.95 | 1.00 | 5.6 | >10 |
| BEA-300 | 0.19 | 1.03 | 0.16 | 0.74 | 0.98 | 0.92 | 3.3 | >10 |

Example 4

A mixture of 49% $ZnCl_2$, 1.3% isosorbide, 2.2% 14&36AG, 0.3% 25AM, 2.2% sorbitol and approximately 45% water (balance) was obtained by dehydration of sorbitol in a concentrated $ZnCl_2$ solution. The color of the solution was black, indicating the presence of an unknown amount of char or tar. The dehydration reaction was stopped at incomplete conversion to obtain the current mixture composition with a high concentration of unreacted sorbitol and 14&36AG.

This mixture was contacted with zeolites MFI-400, USY-80, BEA-300 and the polymer PSDVB, respectively, at several adsorbent to organic content ratios. In Table 3 the results are shown, normalized with respect to the initial concentration to reveal the selectivity of the adsorbents with respect to the various species in the system. Note that in this case the PSDVB sample was used without pretreatment and contained about 50% water. This led to dilution of the mixture as can be seen from the increased water concentration. All adsorbents are able to adsorb the sugar alcohols from the aqueous $ZnCl_2$ solution with very good sugar/$ZnCl_2$ selectivity. PSDVB and BEA-300 show the same trend in affinity: Isosorbide » 1,4-Anhydrosorbitol > Sorbitol >> $ZnCl_2$. This can also be seen from the calculated Isosorbide to 14&36AG and Sorbitol selectivity in Table 2. MFI seems to be a special case. Although some very small amounts of sorbitol and sorbitans appear to adsorbed, the concentration in the solution changes only very little. It appears to only adsorb isosorbide significantly.

This example shows that also high silica USY-80 and the polymer PSDVB showed the same selectivity towards anhydro sugars as found for zeolite BEA-300 and ACW15. This appears a general property of hydrophobic adsorbents.

But this example also shows that MFI breaks the general trend in selectivity for the different anhydro sugars found for hydrophobic adsorbents. Size exclusion of the sorbitans and sorbitol by zeolite MFI is believed to be the reason for this.

*Table 3. Adsorption selectivity of zeolites BEA-300, MFI-400 and polymer PSDVB in a dehydration product solution.*

| Adsorbent | Adsorbent/ solution Ratio g/g | fraction left in solution ($x/x_{in}$) | | | | | Isosorbide selectivity | |
|---|---|---|---|---|---|---|---|---|
| | | $ZnCl_2$ | Isosorbide | 14& 36AG | 25AM | Sorbitol | 14& 36AG | Sorbitol |
| MFI-400 | 0.10 | 1.00 | 0.81 | 0.97 | 0.90 | 0.95 | 5.7 | 3.5 |

(continued)

| Adsorbent | Adsorbent/ solution Ratio g/g | fraction left in solution ($x/x_{in}$) | | | | | Isosorbide selectivity | |
|---|---|---|---|---|---|---|---|---|
| | | $ZnCl_2$ | Isosorbide | 14& 36AG | 25AM | Sorbitol | 14& 36AG | Sorbitol |
| MFI-400 | 0.19 | 1.01 | 0.71 | 0.97 | 0.99 | 0.94 | 8.4 | 4.9 |
| MFI-400 | 0.28 | 0.99 | 0.57 | 0.94 | 0.83 | 0.95 | 6.8 | 8.4 |
| BEA-300 | 0.12 | 1.00 | 0.47 | 0.92 | 0.94 | 0.95 | 6.5 | 9.8 |
| BEA-300 | 0.20 | 1.00 | 0.25 | 0.82 | 0.95 | 0.93 | 4.3 | 11.5 |
| BEA-300 | 0.32 | 1.00 | 0.14 | 0.68 | 0.93 | 0.83 | 2.7 | 5.2 |
| USY-80 | 0.11 | 1.01 | 0.77 | 0.92 | 0.88 | 0.96 | 2.4 | 7.4 |
| USY-80 | 0.21 | 0.97 | 0.63 | 0.85 | 0.83 | 0.95 | 5.7 | 3.5 |
| PS-DVB | 0.041 | 0.97 | 0.84 | 0.92 | 0.89 | 0.96 | 2.1 | 3.9 |
| PS-DVB | 0.091 | 0.93 | 0.63 | 0.81 | 0.85 | 0.86 | 2.0 | 2.7 |
| PS-DVB | 0.129 | 0.90 | 0.50 | 0.74 | 0.77 | 0.83 | 1.9 | 2.9 |

Example 5

The adsorption of sorbitol from an aqueous solution was investigated. A mother solution of 5%w sorbitol in water was contacted with various amounts of MFI-400, BEA-300, USY-80 and PSDVB at 298 K. The results are shown in Table 4. Addition of USY-80, BEA-300 and PSDVB led to a significant decrease of sorbitol in the solution, corresponding to a loading of several %w of sorbitol. However, MFI-400 did not adsorb sorbitol from the solution.

Sorbitol adsorbs on USY-80, BEA-300 and PSDVB because of favorable interactions with the adsorbent surface. MFI-400 was expected to behave similar to zeolite USY-80 and BEA-300, because the materials are similar in composition. This different adsorption properties exhibited by MFI-400 are explained by the difference in pore size between MFI-400 and zeolites BEA-300 and USY-80. MFI-400 has a significantly smaller pore size (Table 1) than zeolite USY-80 and BEA-300. Apparently the pore size of MFI-400 is too small for sorbitol to enter.

This example strongly confirms that sorbitol is not adsorbed on zeolite MFI-400. The mechanism is believed to be size exclusion.

*Table 4. Sorbitol adsorption on various adsorbents from an aqueous solution.*

| Adsorbent | Adsorbent/ solution ratio | Initial Sorbitol fraction | Final Sorbitol fraction | Calculated loading $g/g_{ads}$ |
|---|---|---|---|---|
| MFI-400 | 0.11 | 0.050 | 0.050 | -0.002 |
| MFI-400 | 0.22 | 0.050 | 0.050 | 0.000 |
| MFI-400 | 0.33 | 0.050 | 0.050 | 0.001 |
| USY-80 | 0.22 | 0.050 | 0.045 | 0.021 |
| BEA-300 | 0.21 | 0.050 | 0.047 | 0.016 |
| PSDVB | 0.20 | 0.050 | 0.044 | 0.028 |

Example 6

50 g of a mother solution containing 2,5AM, 1,4&36AG, sorbitol, isosorbide and water was contacted with 25 g of MFI-400 at 298K. The mixture was shaken for 15 minutes. Then the mixture was filtered off (filtrate I). The filtrate (45 g) was contacted with 27 g of fresh MFI-400. Again the mixture was shaken for 15 minutes and filtered (Filtrate II). The compositions of the mother solution, filtrate I and II are shown in Table 5. It is shown that by contacting this mixture with MFI-400 about 90% of all isosorbide is removed from the solution, while leaving virtually all 25AM, 14&36AG and sorbitol in the solution. This example is a strong evidence of the molecular sieving properties of MFI-400.

*Table 5. Weight fractions of a solution before and after contact with zeolite MFI-400.*

| | 25AM | 14&36AG | Isosorbide | Sorbitol |
|---|---|---|---|---|
| Mother solution | 0.0033 | 0.0408 | 0.031 | 0.0010 |
| Filtrate I | 0.0032 | 0.0394 | 0.013 | 0.0008 |

(continued)

| | 25AM | 14&36AG | Isosorbide | Sorbitol |
|---|---|---|---|---|
| Filtrate II | 0.0035 | 0.0384 | 0.003 | 0.0009 |

Example 7

Mixtures of various solvents containing 2%w isosorbide were prepared. The mixtures were contacted with zeolite BEA-300 for 1 h at a BEA-300/isosorbide ratio of 10. The isosorbide mass fraction in the solution before and after contact with BEA-300 are presented in Table 6. Here also the temperature at which the experiments were performed is given.

In water at 298 K the final isosorbide fraction in the solution wais much lower than the initial fraction. Clearly in this case adsorption of isosorbide was favored. In case of water at 363 K, less isosorbide was adsorbed, but still most of the isosorbide was removed from the initial solution. In the presence of most organic solvents very little isosorbide was adsorbed and these solvents are suitable candidates to desorb isosorbide from zeolite BEA-300. Clearly these organic solvents adsorb preferably compared to isosorbide on the hydrophobic zeolite BEA-300.

However, in case of o-xylene and dipropyl ether at 363 K still most of the isosorbide was adsorbed from the solution. This is explained by the very poor solubility of isosorbide in these solvents, hence, still adsorption of isosorbide is favored.

This example shows that a good desorbent for isosorbide will be a solvent with enough affinity with the adsorbent to replace isosorbide and a good isosorbide solubility to promote desorption. Ethanol, methanol and isopropanol are examples of good desorbents to remove isosorbide from zeolite BEA-300.

*Table 6. Isosorbide adsorption on zeolite BEA-300 from various solvents.*

| Solvent | T / K | BEA-300/solution ratio | Initial IS fraction | Final IS fraction |
|---|---|---|---|---|
| Ethanol | 298 | 0.20 | 0.024 | 0.027 |
| Water | 298 | 0.20 | 0.021 | 0.002 |
| Ethanol | 333 | 0.20 | 0.022 | 0.026 |
| Water | 363 | 0.20 | 0.023 | 0.008 |
| o-xylene | 363 | 0.21 | 0.019 | 0.000 |
| Dipropyl ether | 363 | 0.18 | 0.019 | 0.004 |
| Methanol | 298 | 0.20 | 0.022 | 0.019 |
| 1,4-dioxane | 298 | 0.19 | 0.023 | 0.026 |
| Tetrahydrofuran | 298 | 0.19 | 0.024 | 0.028 |
| Methyl ether ketone | 298 | 0.19 | 0.022 | 0.023 |
| Isopropanol | 298 | 0.19 | 0.022 | 0.019 |

Example 8

A solution of 2%w of isosorbide in ethanol were contacted with BEA-300, MFI-400, USY-80, ACW15 and PSDVB at 298K. The isosorbide loadings were calculated as described in the general methods section and listed in Table 7. When these loadings are compared to the isosorbide loadings in an aqueous medium (Figure 1), it is clear that ethanol promotes isosorbide desorption effectively in case of all adsorbents. Note also that also here desorption appears the most effective when the adsorbent has a more micro porous character. This example shows that ethanol as a desorbent is not restricted to zeolite BEA-300, but can be used to desorb isosorbide from a wide variety of hydrophobic adsorbents.

*Table 7. Isosorbide adsorption on various adsorbents from ethanol.*

| Adsorbent | Adsorbent/ solution ratio | Initial IS fraction | Final IS fraction | Calculated loading $g/g_{ads}$ |
|---|---|---|---|---|
| BEA-300 | 0.20 | 0.024 | 0.027 | -0.015 |
| MFI-400 | 0.11 | 0.020 | 0.020 | -0.006 |
| USY-80 | 0.10 | 0.020 | 0.020 | -0.003 |

(continued)

| Adsorbent | Adsorbent/ solution ratio | Initial IS fraction | Final IS fraction | Calculated loading g/$g_{ads}$ |
|---|---|---|---|---|
| ACW15 | 0.10 | 0.023 | 0.021 | 0.0145 |
| PSDVB | 0.10 | 0.023 | 0.019 | 0.038 |

Example 9

Zeolite BEA-300 and activated carbon W15 were contacted with an aqueous solution containing HCl (5%w), Isosorbide (2%w) and Sorbitol (2%w), and with a solution containing $H_2SO_4$ (5%w), Isosorbide (2%w) and Sorbitol (2%w). Table 8 shows the normalized fractions of the solutions after 4 h. The behavior in both solutions was very similar. For both solutions the inorganic acids appeared to be not adsorbed by BEA-300 and slightly by ACW15. BEA-300 adsorbed Isosorbide strongly and Sorbitol only slightly, ACW15 adsorbed both organic components strongly, with a good selectivity towards Isosorbide.
This example demonstrates that isosorbide can be removed selectively using hydrophobic adsorbents not only from a $ZnCl_2$ hydrate but also from aqueous solutions containing other inorganic acids like HCl and $H_2SO_4$. Moreover, using ACW15 also components less hydrophobic than Isosorbide, like sorbitol, can be removed from aqueous inorganic acid solutions.

*Table 8. Adsorption selectivity of ACW15 and zeolite BEA-300 in a HCl and $H_2SO_4$ containing aqueous solution.*

| Adsorbent | Adsorbent/solution Ratio g/g | Fraction left in solution ($x/x_{in}$) | | | |
|---|---|---|---|---|---|
| | | HCl | $H_2SO_4$ | Isosorbide | Sorbitol |
| BEA-300 | 0.099 | 1.01 | n.a. | 0.44 | 0.98 |
| BEA-300 | 0.186 | 1.02 | n.a. | 0.15 | 0.95 |
| ACW15 | 0.101 | 0.97 | n.a. | 0.41 | 0.75 |
| ACW15 | 0.181 | 0.92 | n.a. | 0.36 | 0.68 |
| BEA-300 | 0.097 | n.a. | 1.00 | 0.43 | 1.01 |
| BEA-300 | 0.191 | n.a. | 1.02 | 0.17 | 1.00 |
| ACW15 | 0.101 | n.a. | 0.96 | 0.38 | 0.76 |
| ACW15 | 0.179 | n.a. | 0.93 | 0.18 | 0.51 |

Example 10

A mixture containing isosorbide, isomannide and isoidide was prepared by an isomerization of isosorbide over a Raney Nickel catalyst according to the method of Wright *(*U.S. Patent No. 3023223*)*.

This mother solution was diluted with water to form an aqueous solution with the following composition: 2.0%w Isoidide, 1.4%w Isosorbide and 0.15% Isomannide. The mother solution was contacted with different amounts of zeolite BEA-300, MFI-400 and activated carbon W15. The normalized weight fraction of the components in the solution after 18 h is shown in Table 9.

All three adsorbents showed a very significant adsorption of all three components. In accordance with previous results, the total adsorption capacity of ACW15 was the highest, followed by zeolite BEA-300. In case of BEA-300 and ACW15 all three isomers appeared to have a similar adsorption affinity, and no large selectivity was found. In case of MFI-400 a clear trend in adsorption affinity was found: IM > IS > II. MFI-400 is able to separate Isosorbide from sorbitol and anhydrosorbitol. Apparently the pores of MIF are in the range that Isosorbide can just fit. It is possible that also steric effects contribute to the selective adsorption of the stereo-isomers by MFI-400.

This example shows that the stereo-isomers Isosorbide, Isomannide and Isoidide have a similar adsorption affinity towards zeolite BEA-300 and activated carbon W15 and all of them can therefore be just as easily adsorbed and removed from acid mixtures as demonstrated extensively for isosorbide. However, using MFI-400 these isomers can be also separated from each other.

*Table 9. Adsorption of a mixture of Isomannide, Isoidide and Isosorbide from an aqueous solution on ACW1,5 zeolite BEA-300 and zeolite MFI-400 at 298 K.*

| Adsorbent | Adsorbent/solution Ratio g/g | Fraction left in solution ($x/x_{in}$) | | |
|---|---|---|---|---|
| | | Isoidide | Isosorbide | Isomannide |
| BEA-300 | 0.097 | 0.72 | 0.62 | 0.74 |
| BEA-300 | 0.190 | 0.48 | 0.35 | 0.39 |
| MFI-400 | 0.092 | 0.92 | 0.86 | 0.60 |
| MFI-400 | 0.198 | 0.86 | 0.70 | 0.31 |
| ACW15 | 0.103 | 0.34 | 0.44 | 0.58 |
| ACW15 | 0.194 | 0.10 | 0.16 | 0.16 |

Example 11

Since the high Isomannide/Isosorbide selectivity of MFI-400 demonstrated in the previous example was based on very low Isomannide weight fractions, another series of adsorption experiments was conducted with equal amounts of Isosorbide and Isomannide in the starting solution. In this example a mother solution was prepared containing 1%w Isosorbide and 1%w Isomannide in water. This solution was contacted with different amounts of zeolite MFI-400. Table 10 shows the normalized fractions of the solutions after 4 h together with the adsorption selectivity in each experiment.

This example strongly confirms that Isosorbide and Isomannide can be separated using zeolite MFI-400. The observed selectivities are very high for a separation of stereo-isomers, which can be considered an extremely challenging separation.

*Table 10. Adsorption of a mixture of Isomannide and Isosorbide from an aqueous solution on zeolite MFI-400 at 298 K. Selectivity = $q(IM)/q(IS)*(x_w(IS)/x_w(IM))$.*

| Adsorbent | Adsorbent/solution Ratio g/g | Fraction left in solution ($x/x_{in}$) | | IM/IS - Selectivity |
|---|---|---|---|---|
| | | Isosorbide | Isomannide | |
| MFI-400 | 0.089 | 0.85 | 0.60 | 3.6 |
| MFI-400 | 0.186 | 0.66 | 0.34 | 3.7 |
| MFI-400 | 0.402 | 0.42 | 0.14 | 4.3 |

Example 12

Zeolite BEA-300, zeolite MFI-400 and activated carbon W15 were contacted with an aqueous solution containing $ZnCl_2$ (22%w), Xylitol (2.8%w) and Anhydroxylitols (0.9%w). Note that the term "Anhydroxylitols" refers to a collection of two dehydration products which appear as overlapping peaks in the HPLC chromatogram. This solution was prepared by (partial) dehydration of xylitol in a 50% $ZnCl_2$ solution, and was diluted about two times before use in the adsorption experiment.

Table 11 shows the normalized fractions of the solutions after 4 h. In all cases $ZnCl_2$ appeared not to be adsorbed by BEA-300 and MFI-400, and slightly by ACW15. Zeolite BEA-300 and ACW15 adsorbed both Xylitol and Anhydroxylitols, with a significant preference for the Anhydroxylitols, which can be explained by the fact that the Anhydroxylitols are more hydrophobic than Xylitol. Zeolite MFI-400 adsorbed significant amounts of the Anhydroxylitols but much less than BEA-300 and ACW15. Xylitol appeared to be almost not adsorbed by MFI-400 at all, which could be an indication that this molecule is size-excluded from this zeolite.

This example demonstrates that Xylitol and Anhydroxylitols can be removed from an aqueous $ZnCl_2$ solution, or any other inorganic acid as demonstrated in example 9, using activated carbon (e.g. ACW15) or hydrophobic zeolites (e.g. BEA-300, MFI-400). Moreover, these materials have Anhydroxylitol/Xylitol selectivity and can therefore be used to separate these individual sugar alcohols.

*Table 11. Adsorption selectivity of A CW15, zeolite BEA-300 and zeolite MFI-400 towards (Anhydro)Xylitol in a an aqueous $ZnCl_2$ solution.*

| Adsorbent | Adsorbent/solution Ratio g/g | Fraction left in solution ($x/x_{in}$) | | |
|---|---|---|---|---|
| | | $ZnCl_2$ | Anhydroxylitols | Xylitol |
| ACW15 | 0.10 | 0.98 | 0.48 | 0.72 |
| ACW15 | 0.19 | 0.95 | 0.31 | 0.52 |
| BEA-300 | 0.10 | 1.02 | 0.60 | 0.86 |
| BEA-300 | 0.20 | 1.04 | 0.46 | 0.75 |
| BEA-300 | 0.40 | 1.07 | 0.27 | 0.58 |
| MFI-400 | 0.10 | 1.01 | 0.86 | 1.00 |
| MFI-400 | 0.18 | 1.01 | 0.86 | 0.97 |
| MFI-400 | 0.38 | 1.02 | 0.76 | 0.95 |

Many modifications in addition to those described above may be made to the structures and techniques described herein without departing from the spirit and scope of the invention. Accordingly, although specific embodiments have been described, these are examples only and are not limiting upon the scope of the invention.

Example 13

BEA-300 and activated carbon W15 were contacted with an aqueous solution containing 2%w Glucose and 2%w Cellobiose.

Table 12 shows the normalized fractions of the solutions after 4 h. In case of BEA-300 the fractions of Glucose and Cellobiose are only slightly reduced, indicating a very low amount of adsorption. ACW15 shows a very strong reduction of both Glucose and Cellobiose. Particularly Cellobiose is very strongly adsorbed since almost nothing is left already at a sorbent to solution ratio of 0.10. Clearly ACW 15 has a very high Cellobiose to Glucose selectivity.

This example demonstrates that Glucose and Cellobiose can be adsorbed from an aqueous solution using ACW15. Moreover, Glucose and Cellobiose can be separated from each other using this activated carbon.

*Table 12. Adsorption of ACW15 and CBV 811C-300 towards Glucose and Cellobiose in a an aqueous solution at 298K.*

| Adsorbent | Adsorbent/solution Ratio g/g | Fraction left in solution | |
|---|---|---|---|
| | | Glucose | Cellobiose |
| | 0 | 0.020 | 0.020 |
| BEA-300 | 0.09 | 0.020 | 0.020 |
| BEA-300 | 0.19 | 0.020 | 0.019 |
| BEA -300 | 0.38 | 0.019 | 0.018 |
| ACW15 | 0.10 | 0.015 | 0.001 |
| ACW15 | 0.20 | 0.007 | 0.000 |
| ACW15 | 0.26 | 0.011 | 0.000 |

Example 14

An all-silica MFI zeolite membrane is synthesized according to the method of Vroon et al. (Z.A.E.P. Vroon, Synthesis and transport properties of thin ceramic supported zeolite (MFI) membranes, Ph.D. dissertation, University of Twente, The Netherlands, 1995 (Chapters II and III). As a support an $\alpha$-alumina disc with a diameter of 20 mm is used. The resulting membrane has a 2 micrometer MFI top layer. The membrane is contacted with a feed and a sweep flow of 1 ml $min^{-1}$ at 333 K. The corresponding outgoing flows are called the retentate and permeate flows, respectively. The zeolite layer faces the feed side. The feed solution consists of the following components by weight: 0.5 ($ZnCl_2$), 0.43 (Water), 0.05 (Isosorbide), 0.01 (Sorbitol), 0.01 (Sorbitans). As a sweep liquid, pure water is used to reduce water permeation from the feed to the permeate side. The sweep side pressure is fixed at 101 kPa, the feed side is set to 200 kPa to balance the water permeation from the sweep to the feed side.

From the weight fractions of the individual components in the outgoing flows the Isosorbide selectivity with respect to the different components is calculated as follows:

$$Selectivity_{IS,i} = \left(\frac{x_{IS}}{x_i}\right)^{Permeate} \left(\frac{x_i}{x_{IS}}\right)^{Retentate}$$

The MFI zeolite membrane has Isosorbide/$ZnCl_2$; Isosorbide/Sorbitans; and Isosorbide/Sorbitol selectivities that are high enough to allow selective removal of Isosorbide from a mixture containing $ZnCl_2$, Sorbitol, Sorbitans and water using a MFI zeolite membrane.

## Claims

1. A method for recovering anhydrosugar alcohols from a mixture further containing sugar alcohols, said method comprising the steps of:

   a. Contacting the mixture with an adsorbent, whereby the anhydrosugar alcohols are selectively adsorbed;
   b. Separating the adsorbent from the mixture;
   c. Desorbing anhydrosugar alcohols from the adsorbent by contacting the adsorbent with a desorbing solvent.

2. The method of claim 1 comprising the further step of:

   d. isolating the anhydrosugars alcohols from the desorbing solvent.

3. The method of claim 1 or 2 wherein the selective adsorption of step a. is based on size exclusion.

4. The method of claim 3 wherein the adsorbent is a molecular sieve.

5. The method of claim 4 wherein the adsorbent is zeolite MFI.

6. The method of claim 4 or 5 wherein the adsorbent is in the form of a zeolite membrane, and the desorbing solvent is used as sweep liquid.

7. The method of claim 1 or 2 wherein the selective adsorption of step a. is based on hydrophobicity.

8. The method of claim 7 wherein the adsorbent is selected from the group consisting of activated carbon and hydrophobic zeolites.

9. The method of any one of the preceding claims wherein the desorbing solvent comprises one or more alkanols having from 1 to 4 carbon atoms.

10. The method of any one of the preceding claims wherein the anhydrosugar alcohols comprise anhydroxylitols; and the sugar alcohols comprise xylitol.

11. The method of any one of the preceding claims wherein the anhydrosugars alcohols comprise isosorbide, and the sugar alcohols comprise sorbitol.

12. The method of any one of the preceding claims wherein the mixture further comprises an inorganic acid.

13. The method of claim 9 wherein the inorganic acid comprises a molten salt hydrate.

14. The method of claim 10 wherein the molten salt hydrate comprises $ZnCl_2$.

15. The method of claim 12 comprising the steps of:

   a. Contacting the mixture comprising the inorganic acid with a first adsorbent whereby the organic components are selectively adsorbed;
   b. Separating the first adsorbent from the mixture;
   c. Desorbing the organic components from the first adsorbent by contacting the first adsorbent with a first

desorbing solvent, to obtain a mixture comprising sugar alcohols and anhydrosugars alcohols;

d. Contacting the mixture obtained in step c. with a second adsorbent, whereby the anhydrosugar alcohols are selectively adsorbed;

e. Separating the second adsorbent from the mixture;

f. Desorbing anhydrosugar alcohols from the second adsorbent by contacting the second adsorbent with a second desorbing solvent.

**16.** The method of claim 12 comprising the steps of:

a. Contacting the mixture comprising the inorganic acid with a first adsorbent whereby the anhydrosugar alcohols are selectively adsorbed;

b. Separating the mixture from the first adsorbent;

c. Contacting the mixture obtained in step b. with a second adsorbent, whereby the sugar alcohols are selectively adsorbed.

**17.** The method of claim 15 or 16 wherein the first adsorbent is identical to the second adsorbent.

**18.** The method of claim 15 or 16 wherein the first adsorbent is different from the second adsorbent.

**19.** The method of claim 15 wherein the first adsorbent comprises activated carbon.

**20.** The method of claim 16 wherein the second adsorbent comprises activated carbon.

Figure 1A

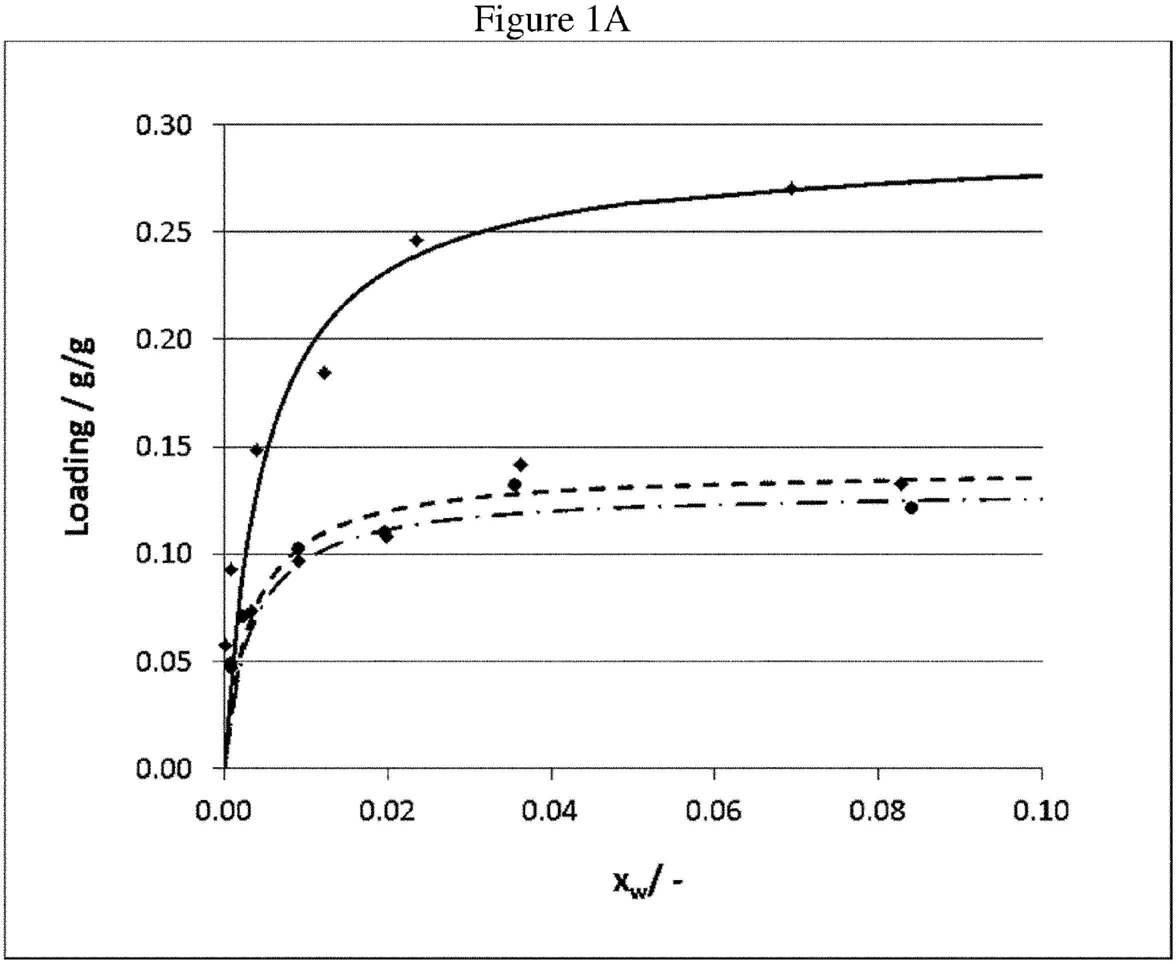
0.30
0.25
0.20
0.15
0.10
0.05
0.00
Loading / g/g
0.00
0.02
0.04
0.06
0.08
0.10
$x_w$/ -

Figure 1B

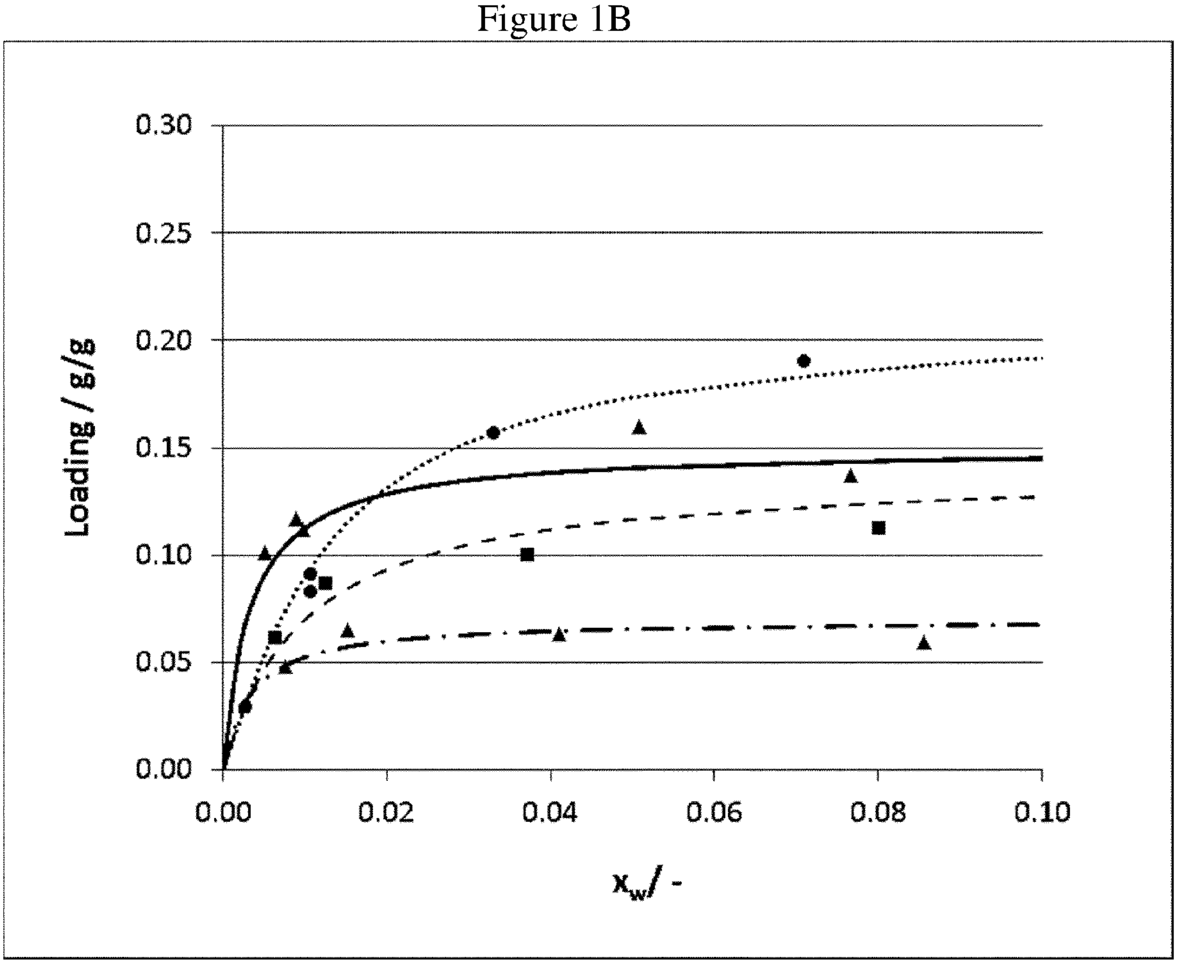
0.30
0.25
0.20
0.15
0.10
0.05
0.00
Loading / g/g
0.00
0.02
0.04
0.06
0.08
0.10
$x_w$/ -

Figure 2A

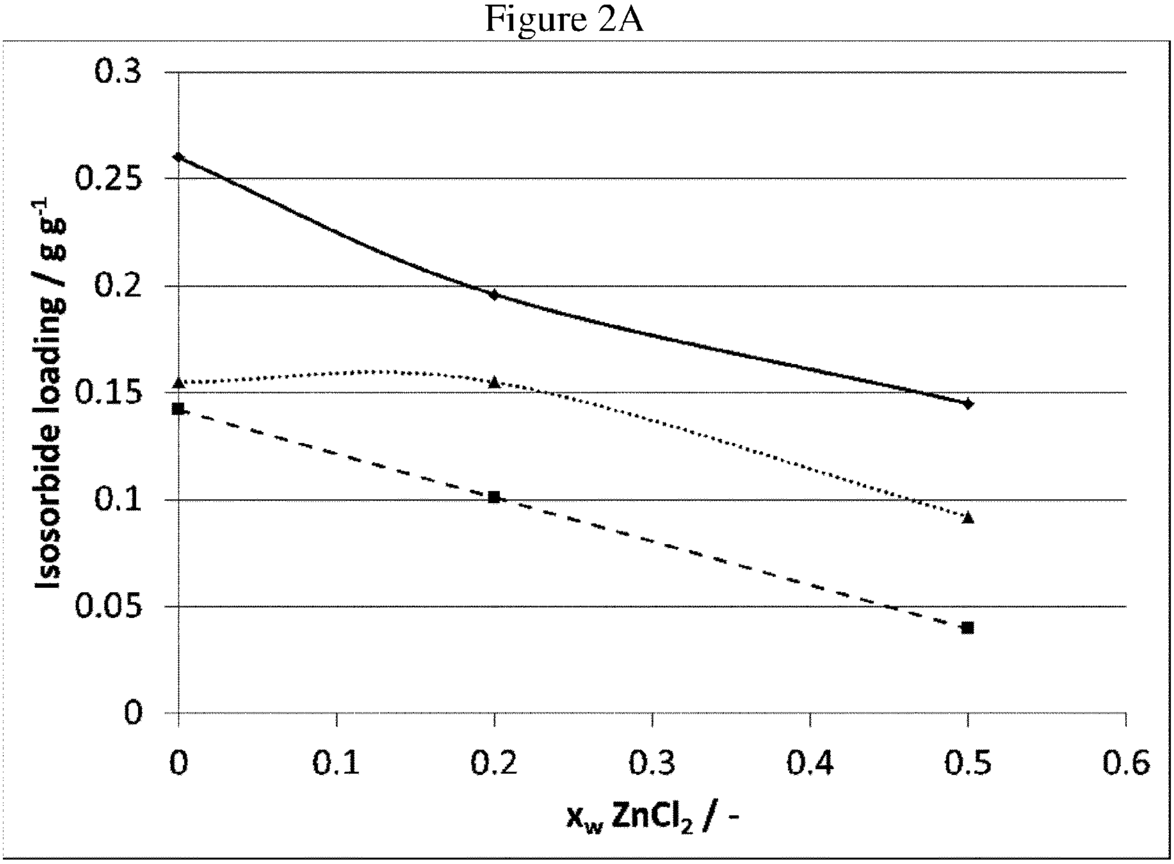
0.3
0.25
0.2
0.15
0.1
0.05
0
Isosorbide loading / g g-1
0
0.1
0.2
0.3
0.4
0.5
0.6
xw ZnCl2 / -

Figure 2B

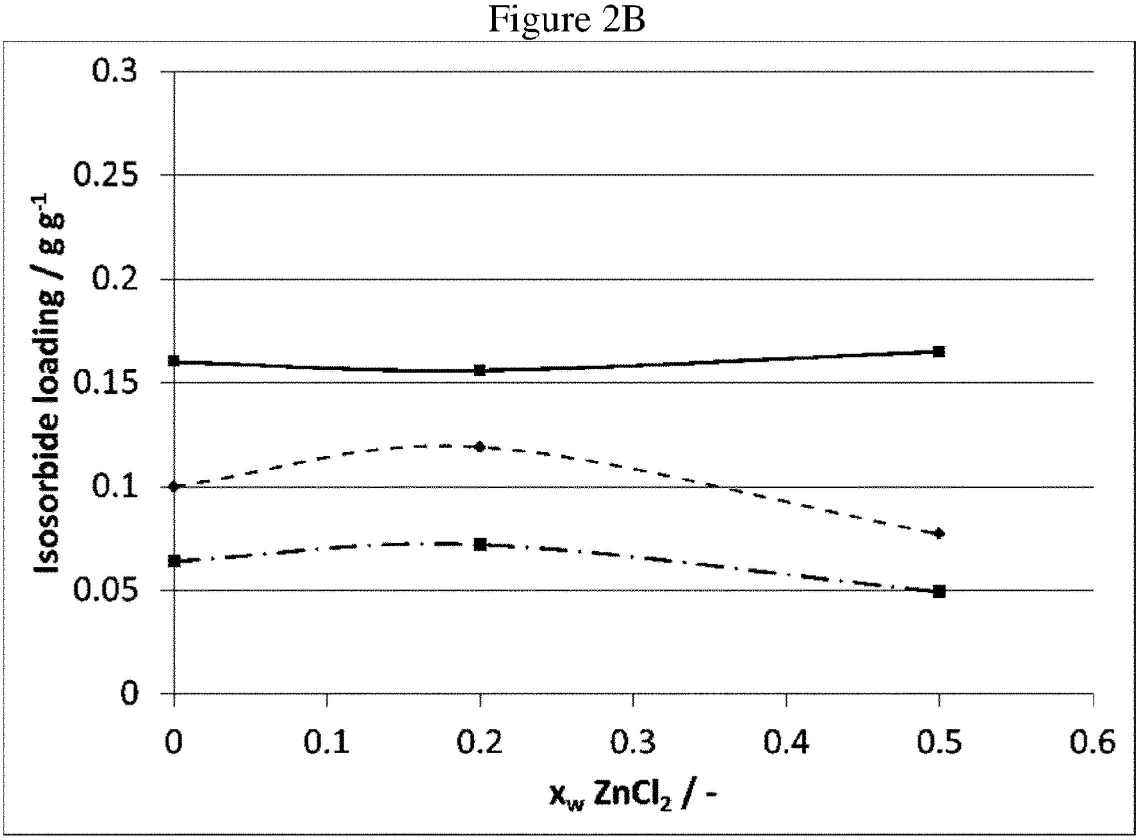
Isosorbide loading / g g-1
0.3
0.25
0.2
0.15
0.1
0.05
0
0
0.1
0.2
0.3
0.4
0.5
0.6
xw ZnCl2 / -

Europäisches Patentamt
European Patent Office
Office européen des brevets

# EUROPEAN SEARCH REPORT

Application Number
EP 12 15 1277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/39957 A2 (DU PONT [US]; DALZIEL SEAN MARK [US]; GREEN DANIEL ALBERT [US]; ZOLAND) 23 May 2002 (2002-05-23)<br>* the whole document *<br>----- | 1-20 | INV.<br>C07D493/04 |
| A | US 2005/202141 A1 (CORBIN DAVID R [US] ET AL CORBIN DAVID RICHARD [US] ET AL) 15 September 2005 (2005-09-15)<br>* the whole document *<br>----- | 1-20 | |
| A | WO 99/18094 A1 (HENKEL CORP [US]) 15 April 1999 (1999-04-15)<br>* the whole document *<br>----- | 1-20 | |
| | | | TECHNICAL FIELDS SEARCHED (IPC) |
| | | | C07D |

The present search report has been drawn up for all claims

1

| Place of search | Date of completion of the search | Examiner |
|---|---|---|
| Munich | 10 May 2012 | Nikolai, Joachim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 1277

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2012

| Patent document cited in search report | Publication date | Patent family member(s) | Publication date |
|---|---|---|---|
| WO 0239957 A2 | 23-05-2002 | AU 3516602 A | 27-05-2002 |
| | | WO 0239957 A2 | 23-05-2002 |
| US 2005202141 A1 | 15-09-2005 | NONE | |
| WO 9918094 A1 | 15-04-1999 | AU 744457 B2 | 21-02-2002 |
| | | AU 9474098 A | 27-04-1999 |
| | | BR 9812725 A | 22-08-2000 |
| | | CA 2305197 A1 | 15-04-1999 |
| | | CN 1274351 A | 22-11-2000 |
| | | EP 1032567 A1 | 06-09-2000 |
| | | TR 200000917 T2 | 21-07-2000 |
| | | TW 467905 B | 11-12-2001 |
| | | US 6680392 B1 | 20-01-2004 |
| | | WO 9918094 A1 | 15-04-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6864378 B, Bhatia
- US 7439352 B, Moore
- WO 0014081 A
- US 4564692 A, Feldmann
- US 2524414 A, Wolfrom
- WO 0239957 A2
- US 20050202139 A1
- US 20050202141 A1
- US 3023223 A


### Non-patent literature cited in the description


- **ALMEIDA et al.** *ChemSusChem,* 2010, vol. 3, 325
- Z.A.E.P. Vroon, Synthesis and transport properties of thin ceramic supported zeolite (MFI) membranes. **VROON et al.** Ph.D. dissertation. 1995